# EUROPÄISCHE PATENTSCHRIFT

(11) **EP 1 024 760 B1**
(45) Veröffentlichungstag und Bekanntmachung des Hinweises auf die Patenterteilung: **08.12.2004**
(21) Anmeldenummer: 98955517.2
(22) Anmeldetag: 23.10.1998
(51) Int. Cl.: A61B 18/00, G02B 6/42, G02B 6/38

(54) **MEDIZINISCHES HANDSTÜCK MIT IN AXIALER RICHTUNG VERSCHIEBBAREM LICHTLEITER**
MEDICAL HANDPIECE WITH A LIGHT GUIDE WHICH CAN BE DISPLACED IN AN AXIAL DIRECTION
PIECE A MAIN A USAGE MEDICAL, AVEC GUIDE DE LUMIERE DEPLA ABLE EN SENS AXIAL

(30) Priorität: 24.10.1997 DE 19747046
(43) Veröffentlichungstag der Anmeldung: 09.08.2000
(73) Patentinhaber: Carl Zeiss Meditec AG, 07745 Jena (DE)
(72) Erfinder: ELBRECHT, Jens, D-07747 Jena (DE); KALVE, Berlind, D-07548 GERA (DE); KLOSS, Thomas, D-07743 Jena (DE)
(74) Vertreter: Niestroy, Manfred
(86) Internationale Anmeldenummer: PCT/EP1998/006736
(87) Internationale Veröffentlichungsnummer: WO 1999/021493

(56) Entgegenhaltungen:
- EP-A- 0 507 991
- DE-A- 4 126 886
- US-A- 4 694 828
- US-A- 4 785 805
- US-A- 5 825 958
- US-A- 5 957 914

## Beschreibung

Die Erfindung bezieht sich auf ein medizinisches Handstück zur Übertragung von Energie aus einer Laserstrahlung in biologisches Gewebe, bei dem in einem Grundkörper eine optische Faser zur Heranführung der Laserstrahlung und ein Lichtleiter zur Abstrahlung der Laserenergie in das Gewebe angeordnet sind.

### Stand der Technik

Die bekannten Handstücke dieser Art weisen eine feste Verbindung zwischen dem Lichtleiter und dem Grundkörper des Handstückes auf. Dabei hat sich gezeigt, daß die Lichtleiter, die in der Regel aus Quarz oder Saphir bestehen, nach ca. vier Operationen verschleißen. Der Grund liegt darin, daß das polierte Faserende welches in Kontakt mit dem Gewebe gebracht wird, sich aufrauht und der Abtrag uneffektiv wird. Dies tritt vor allem bei hoher Laserimpulsfolge auf und resultiert wahrscheinlich aus der Bildung von Kavitationsblasen.

Es ist von Nachteil, daß sich der Lichtleiter nicht wechseln läßt. So muß der gesamte Applikator ausgetauscht werden, wenn der Lichtleiter verschlissen ist. Nach Ende der möglichen Nutzdauer ist wegen der unlösbaren Verbindung also nicht nur der Lichtleiter verloren, sondern mit diesem auch der Grundkörper und weitere funktionswesentliche Teile des Handstückes.

Ein weiterer Nachteil besteht darin, daß sich das Handstück aufgrund der sehr engen Zwischenräume zwischen den Bauteilen im kompletten Zustand schlecht reinigen läßt. So muß der Raum zwischen der Kanüle (Durchmesser kleiner 1 mm) und dem Lichtleiter (Durchmesser größer 0,4 mm) hygienisch einwandfrei gereinigt werden; ein demontierbares Handstück wäre hier wünschenswert, setzt aber eine gute Justierbarkeit der funktionswesentlichen Baugruppen voraus.

In US 4,785,805 ist eine Vorrichtung zur Einkopplung einer von ihrer Quelle kommenden Laserstrahlung in einen Lichtleiter beschrieben, bei der dem Lichtleiter eine Sammellinse vorgeordnet ist. Dieser Vorrichtung sind Hinweise auf die technischen Mittel zu entnehmen, mit denen eine Zentrierung der optischen Achsen von Sammellinse und Lichtleiter zueinander als Voraussetzung für eine möglichst verlustfreien Einkopplung erreicht wird. Allerdings besteht hierbei nicht die Notwendigkeit und es sind auch keine technischen Mittel dafür vorgesehen, die optischen Achsen von Sammellinse und Lichtleiter auch noch zu einer Absaugkanüle auszurichten, innerhalb derer der Lichtleiter in definierter Lage zu verlaufen hat, so wie dies in einem Handstück der eingangs genannten Art der Fall ist.

In EP 0 507 991 ist ein Adapter zur Lichtleitereinkopplung für ein Lasergerät beschrieben. Der Adapter enthält ein hülsenförmiges Paßteil zu Aufnahme des proximalen Lichtleiterendes und ist so ausgebildet, daß im Bereich der Verbindungsstelle zwischen dem Paßteil und einem Griff Sperr- oder Koppelmittel vorgesehen sind, durch welche die Verbindung von Paßteil und Griff erst bei eingefügtem Lichtleiter ermöglicht wird. Ein Lichtleiter zur Abstrahlung der Laserenergie in beispielhaft biologisches Gewebe, der dem Verschleiß unterworfen ist und deshalb häufig gewechselt und jeweils beim Wechsel zu einem weiteren Lichtleiter und auch noch zu einer Absaugkanüle, in der er verläuft, auszurichten ist, ist hierbei nicht vorgesehen.

Das trifft analog auch auf den Spülkatheter nach DE 41 26 886 A1 zu. Dieser dient der Beseitigung von Feststoffen aus Körperorganen und Körperhohlorganen von Menschen und Tieren und ist mit mindestens zwei Lumen versehen, von denen eines der Zufuhr eines Spülfluids von einer Hochdruckfluidquelle in das betreffende Organ dient und das zweite Lumen zur Abfuhr des Spülfluids und dem vom Spülfluid mitgenommenen Feststoffteilchen aus dem Organ vorgesehen ist.

Hier jedoch ist weder eine Energieübertragung mittels Laser über Lichtleiter noch eine Um- oder Einkopplung dieser Energie vorgesehen, so daß auch mit den in dieser Offenlegung angegebenen Mitteln das Problem, einer weitestgehend selbsttätige Ausrichtung zweier Lichtleiter bezüglich ihrer optischen Achsen und auch bezüglich einer Absaugkanüle bei Montage nach einer Reinigung bzw. nach dem Wechseln eines der Lichtleiter zu erreichen, nicht gelöst ist.

Ein Handstück gemäß Oberbegriff von Anspruch 1 ist aus US-5 825 958 bekannt.

### Beschreibung der Erfindung

Aufgabe der Erfindung besteht darin, ein unkompliziertes Auswechseln des Lichtleiters zu ermöglichen und außerdem bei geringem Demontage- und Montageaufwand eine einwandfreie, allen hygienischen Anforderungen genügende Reinigung zu gewährleisten. Diese Aufgabe wird durch die Merkmale des Anspruchs 1 gelöst.

Dazu ist bei einem medizinischen Handstück der eingangs beschriebenen Art vorgesehen, daß zwecks Ausrichtung der optischen Achse des Lichtleiters sowohl in Bezug auf die Längsachse der Absaugkanüle als auch in Bezug auf die optische Achse des abstrahlungsseitigen Endes der Faser mindestens je eine Justierfläche dem Lichtleiter, der Absaugkanüle und der Faser ortsunveränderlich zugeordnet sind, wobei mindestens je eine der dem Lichtleiter zugeordneten Justierflächen mit einer der Absaugkanüle und mit einer der Faser zugeordneten Justierfläche korrespondiert und wobei der Lichtleiter relativ zum Grundkörper in axialer Richtung verschiebbar und die Ausrichtung in Abhängigkeit von der Verschieberichtung herstellbar oder aufhebbar ist.

Mit diesem Aufbau ergibt sich der wesentliche Vorteil, daß der Lichtleiter, der zur Abstrahlung der Laserenergie in das Gewebe hinein dient, von der optischen Faser, mit welcher die Laserstrahlung von der Laserquelle zum Handstück geführt wird, getrennt ist und mit geringem Aufwand aus dem Handstück entnommen und gegen einen unverbrauchten Lichtleiter ausgetauscht werden kann. Die Entnahme des Lichtleiters aus dem Grundkörper des Handstücks ist durch Verschiebung in axialer Richtung entgegengesetzt zur Strahlungsrichtung der Laserstrahlung möglich. Umgekehrt kann das Einsetzen des neuen Lichtleiters durch Einführung und Vorschieben in axialer Richtung in den Grundkörper des Handstücks erfolgen, wobei eine selbsttätige Ausrichtung des Lichtleiters innerhalb einer Absaugkanüle erfolgt, durch die das unter Energieeinwirkung zerkleinerte biologische Gewebe von der Behandlungsstelle abgesaugt wird. Ebenso erfolgt die Ausrichtung der optischen Faser relativ zum Grundkörper des Handstücks und damit auch zugleich zum Lichtleiter, während das optische Ende der Faser in das Handstück hineingeschoben und dabei zentriert wird.

Damit ist gewährleistet, daß der Lichtleiter nach der Montage bzw. nach Austausch eines verbrauchten Lichtleiters mit wenigen Handgriffen innerhalb des Handstückes und damit auch innerhalb der Absaugkanüle die vorgesehene, für den Erfolg der medizinischen Behandlung notwendige Position einnimmt. Auf den Querschnitt der Absaugkanüle bezogen, der vorzugsweise kreisrund ausgebildet ist, kann der ebenfalls mit kreisrundem Querschnitt ausgeführte Lichtleiter zentrisch angeordnet sein. Eine vorteilhafte Alternative besteht jedoch darin, daß der Lichtleiter versetzt zum Zentrum der Absaugkanüle angeordnet ist, so daß eine größere Querschnittsfläche verbleibt, durch welche die bei der Behandlung anfallenden Partikel absaugbar sind.

Zum Zweck der Ausrichtung sind sowohl dem Grundkörper als auch dem Lichtleiter Justierflächen zugeordnet, wobei die Justierung dann vollzogen ist, wenn sich diese Justierflächen unmittelbar berühren. Diese Justierflächen können alternativ als kreisrunde Zylinderflächen ausgebildet sein, die eine Gleitpassung bilden und dadurch in axialer Richtung gegeneinander verschiebbar sind, oder es ist die Ausbildung der Justierflächen als Kreiskegelflächen mit selbstzentrierender Neigung vorgesehen. Dabei ist jeweils eine der Flächen, gleichgültig ob Zylinder- oder Kreiskegelfläche, als Innenfläche, die gegenüberliegende als Außenfläche gestaltet. Weiterhin haben die Zentrierflächen jeweils kreisrunden Querschnitt. In axialer Richtung ist die Bewegungsfreiheit durch Anschläge begrenzt. Dabei kann vorgesehen sein, daß zwischen den Anschlägen eine mechanische Feder oder ein Körper aus elastischem Material eingeordnet ist, wobei durch die Verschiebung in axialer Richtung eine Vorspannkraft erzeugt und durch das Ausmaß der Verschiebung in axialer Richtung die Vorspannkraft erhöht oder verringert werden kann. Weiterhin können zwischen den aneinandergrenzenden Abschnitten der Anschlagflächen Dichtungselemente vorgesehen sein.

Die Erfindung wird nachfolgend anhand eines Ausführungsbeispieles näher erläutert.

### Kurze Beschreibung der Zeichnung

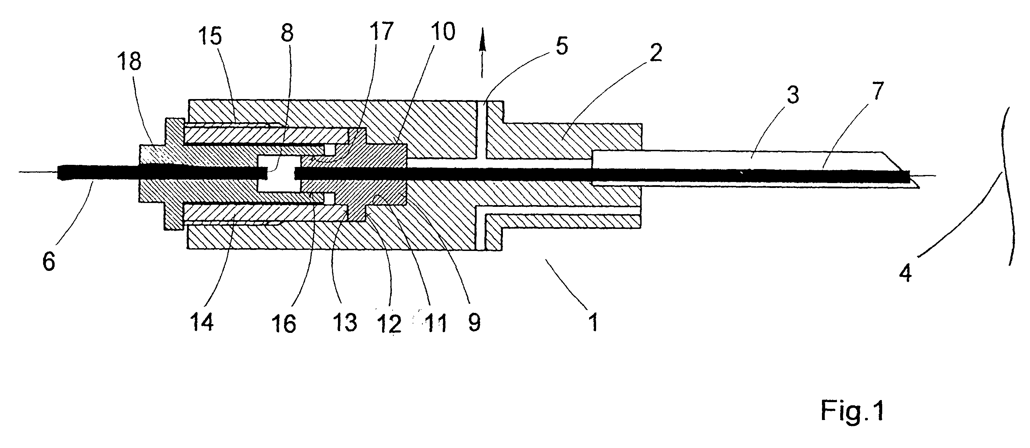

Fig. 1 zeigt eine Prinzipdarstellung des erfindungsgemäßen Handstückes im Querschnitt.

### Ausführliche Beschreibung der Zeichnung

In Fig.1 ist ein Handstück 1 zur Übertragung von Energie aus einer IR-Laserstrahlung in biologisches Gewebe dargestellt, wobei das biologische Gewebe abladiert wird. Mit dem Grundkörper 2 ist eine Absaugkanüle 3 fest verbunden, durch die das unter Energieeinwirkung zerkleinerte biologische Gewebe vom Behandlungsort 4 abgesaugt wird. Die Absaugung erfolgt durch die Absaugkanüle 3 und durch den Absaugkanal 5 hindurch, der in den Grundkörper 2 eingearbeitet ist.

Zur Heranführung der IR-Laserstrahlung ist eine optische Faser 6 vorgesehen, und innerhalb der Absaugkanüle 3 ist ein Lichtleiter 7, bestehend aus Quarz oder Saphir, angeordnet.

Um eine sichere Funktionsweise des medizinischen Handstückes 1 zu gewährleisten, ist es erforderlich, den Lichtleiter 7 in eine definierte Position zum Grundkörper 2 und zu der an dem Grundkörper 2 befestigten Absaugkanüle 3 zu bringen und dort zumindest während der Behandlung sicher zu halten. Außerdem ist es erforderlich, die optischen Achsen des Lichtleiters 7 und der optischen Faser 6 so zueinander zu justieren, daß eine möglichst verlustfreie Umkopplung der Strahlungsenergie von der optischen Faser 6 in den Lichtleiter 7 möglich ist.

Ausgehend von der Aufgabe der Erfindung, die darin besteht, die vorgenannten Forderungen zu erfüllen und außerdem ein unkompliziertes Auswechseln des relativ schnellen verschleißenden Lichtleiters 7 zu ermöglichen wie auch eine einwandfreie, allen hygienischen Anforderungen genügende Reinigung zu ermöglichen, sind erfindungsgemäß Mittel zur Ausrichtung der optischen Achse des Lichtleiters 7 in Bezug auf die Längsachse der Absaugkanüle 3 sowie auch Mittel zur Ausrichtung der optischen Achse des Lichtleiters 7 in Bezug auf die optische Achse des abstrahlungsseitigen Endes 8 der optischen Faser 6 vorgesehen.

Um nun zu gewährleisten, daß der Lichtleiter 7 auf einfache und unkomplizierte Weise aus dem Grundkörper 2 entfernt und gegen einen unverbrauchten ausgetauscht werden kann, sind die Mittel zur Ausrichtung der optischen Achse des Lichtleiters 7 so ausgebildet, daß der Lichtleiter 7 relativ zum Grundkörper 2 bzw. zur Absaugkanüle 3 in axialer Richtung beweglich ist, wobei mit dieser Bewegung in Strahlungsrichtung die Ausrichtung des Lichtleiters 7 in Bezug auf die Absaugkanüle 3 vorgenommen wird, bei Bewegung in entgegengesetzter Richtung, d.h. entgegengesetzt der Strahlungsrichtung der Laserstrahlung, diese Ausrichtung aufgehoben wird.

Um das zu erreichen, ist dem Lichtleiter 7 über ein erstes justierstück 9, das kraft- und/oder formschlüssig, auf jeden Fall fest mit dem Lichtleiter 7 verbunden ist, eine Justierfläche 10 zugeordnet. Die Justierfläche 10 ist als Zylindermantelfläche bzw. Zylinderaußenfläche am Justierstück 9 ausgebildet. Beispielsweise ist die Zylinderachse parallel bzw. zentrisch zur optischen Achse des Lichtleiters 7 ausgerichtet.

Am Grundkörper 2 ist eine Justierfläche 11 vorgesehen, die als Zylinderinnenfläche ausgebildet ist und die mit der Justierfläche 10 korrespondiert. Die beiden Justierflächen 10 und 11 bilden miteinander eine Gleitpassung, wodurch sie in axialer Richtung gegeneinander verschiebbar sind.

Alternativ zu der Ausbildung der Justierflächen 10 und 11 als Kreiszylinderflächen kann vorgesehen sein, diese Justierflächen als Kreiskegelflächen auszubilden. Die Verjüngungsrichtung der beiden Kreiskegelflächen sollte dabei identisch sein mit der Richtung der Laserstrahlung, so daß das Justierstück 9, an dem sich in diesem Fall die als Außenfläche ausgebildete Kreiskegelfläche befindet, durch Bewegung in axialer Richtung entgegengesetzt zur Laserstrahlung von der Justierfläche 11 bzw. aus der ausgerichteten Position und aus dem Grundkörper 2 heraus entfernen läßt.

Beim Einfügen des Lichtleiters 7 mit dem Justierstück 9 in Richtung der optischen Achse erfolgt dagegen eine Selbstzentrierung der beiden Kreiskegelflächen zueinander. Analog ist das ebenfalls so bei der Ausbildung der Justierflächen 10 und 11 als Zylindermantelflächen. Dabei ist sogar eine (in der Verschiebeweite begrenzte) Verschiebung des Lichtleiters 7 einschließlich des Justierstückes 8 in axialer Richtung möglich, ohne daß dabei sogleich die Ausrichtung des Lichtleiters 7 zur Ansaugkanüle 3 aufgehoben wird.

Um zu verhindern, daß eine unbeabsichtigte Verschiebung des Lichtleiters 7 zusammen mit dem Justierstück 9 während der medizinischen Behandlung erfolgt, sind Anschlagflächen 11 und 13 vorgesehen, zwischen denen das Justierstück 9 (in axialer Richtung) unbeweglich eingeschlossen ist. Die Anschlagflächen 12 und 13 sind dabei an verschiedenen Körpern angeordnet, die zwar aneinander befestigt werden können, aber nach Lösung voneinander frei beweglich sind. So ist die Anschlagfläche 12 am Grundkörper 2 ausgebildet, während die Anschlagfläche 13 an einer Buchse 14 vorgesehen ist.

Die Buchse 14 kann, wie das beispielhaft in Fig.1 dargestellt ist, über ein Gewinde 15 mit dem Grundkörper 2 verbunden sein. Bei Einschraubbewegung der Buchse 14 in Richtung der IR-Laserstrahlung bis zur Anlage der Anschlagflächen 12 und 13 am Justierstück 9 erfolgt eine Klemmung des Justierstückes 9 in axialer Richtung, wodurch seine Positionsänderung in axialer Richtung während der Benutzung des medizinischen Handstückes 1 verhindert ist. Wird die Buchse 14 dagegen durch Drehung in entgegengesetzte Richtung gelöst und aus den Grundkörper 2 entfernt, ist das Justierstück 9 mit dem Lichtleiter 7 in axialer Richtung entgegengesetzt zur Strahlungsrichtung frei beweglich und kann ohne weiteres aus den Grundkörper 2 entnommen und gegen ein neues Justierstück 9 mit daran befestigtem Lichtleiter 7 ersetzt werden.

Um nun auch auf ebenso einfache Weise die Ausrichtung der optischen Achse der Faser 6 zur optischen Achse des Lichtleiters 7 bewerkstelligen zu können, ist das Justierstück 9 mit einer weiteren Justierfläche 16 ausgestattet. Die Justierfläche 16 ist ebenfalls als Außenfläche eines Kreiszylinders ausgebildet. Sie korrespondiert mit einer Justierfläche 17, die an einem zweiten Justierstück 18 angeordnet ist. Das zweite Justierstück 18 ist mechanisch fest mit dem abstrahlungsseitigen Ende 8 der optischen Faser 6 verbunden. Die Buchse 14 weist nun eine zentrische Ausnehmung auf, durch welche das Justierstück 18 soweit eingeführt ist, bis die beiden Justierflächen 16 und 17 sich in Kontakt miteinander befinden und dabei eine Ausrichtung der optischen Achse der Faser 6 zur optischen Achse des Lichtleiters 7 erfolgt ist.

Auch die Justierflächen 16 und 17 können alternativ zur dargestellten Ausbildung als Kreiszylinderflächen als Kreiskegelflächen gestaltet sein, wobei jedoch die Verjüngungsrichtung dieser beiden Kegelflächen entgegengesetzt zur IR-Laserstrahlungsrichtung ausgeführt sein sollte. Damit ist es leicht möglich, bei Bewegung des Justierstückes 1 8 entgegengesetzt zur IR-Laserstrahlungsrichtung der beiden optischen Achsen von Lichtleiter 7 und Faser 6 aufzuheben bzw. diese Ausrichtung herzustellen, indem das Justierstück 18 in Richtung der Laserstrahlung in die Ausnehmung der Buchse 14 so weit eingeführt wird, bis sich durch Überginanderschieben der in diesem Fall kegelförmig ausgebildeten Justierflächen 16 und 17 die Zentrierung bzw. Ausrichtung der beiden optischen Achsen der Faser 6 und des Lichtleiters 7 ergibt.

Das erfindungsgemäße Handstück ermöglicht eine schnelle Demontage der einzelnen Baugruppen bzw. Teile zum Zweck der Auswechslung des Lichtleiters 7, wobei die Auswechslung dem Austausch verschlissener Lichtleiter 7, aber auch dem Austausch von Lichtleitern 7 mit unterschiedlicher, aus der Absaugkanüle 3 herausragender oder bereits innerhalb der Absaugkanüle 3 endender Länge dienen kann. Außerdem ist eine sehr rasche Demontage zum Zweck der klinischen Reinigung möglich.

Alternativ zu der hier dargestellten Ausgestaltung der Erfindung mit in axialer Richtung unbeweglich geklemmtem Justierstück 9 und damit in axialer Richtung unbeweglichem Lichtleiter 7 kann vorgesehen sein, daß zwischen der Anschlagfläche 13 an der Buchse 14 und der korrespondierenden Anschlagfläche am Justierstück 9 eine mechanische Feder oder ein elastischer Körper vorgesehen ist, durch welche das Justierstück 9 unter elastischer Vorspannung gegen den Grundkörper 2 gehalten wird. Wird nun manuell eine Kraft auf den Lichtleiter 7 mit entgegengesetzt zur Laserstrahlung ausgerichteter Wirkungsrichtung ausgeübt, ist eine Positionsänderung des Lichtleiters 7 mit dem Justierstück 9 in axialer Richtung in begrenztem Maße möglich. Durch die Kraftwirkung muß dabei lediglich die Vorspannkraft der Feder bzw. des elastischen Körpers überwunden werden.

Daraus ergibt sich vorteilhaft die Möglichkeit, die Abstände zwischen dem Ende der Absaugkanüle 3, dem abstrahlungsseitigen Ende des Lichtleiters 7 und dem Behandlungsort 4 zu variieren.

## Patentansprüche

1. Medizinisches Handstück (1) zur Übertragung von Energie aus einer Laserstrahlung in biologisches Gewebe, bei dem in einem Grundkörper (2) eine optische Faser (6) zur Heranführung der Laserstrahlung, ein Lichtleiter (7) zur Abstrahlung der Laserenergie in das Gewebe sowie eine Absaugkanüle (3) für das unter Energieeinwirkung zerkleinerte biologische Gewebe, in welcher der Lichtleiter (7) verläuft, vorgesehen sind, und
- zwecks Ausrichtung der optischen Achse des Lichtleiters (7) sowohl in Bezug auf die Längsachse der Absaugkanüle (3) als auch in Bezug auf die optische Achse des abstrahlungsseitigen Endes (8) der Faser (6) mehrere Justierflächen (10, 11, 16, 17) vorhanden sind,
**dadurch gekennzeichnet, daß**
- die Absaugkanüle (3) mit dem Grundkörper (2), der Lichtleiter (7) mit einem ersten Justierstück (9) und die optische Faser (6) mit einem zweiten Justierstück (18) mechanisch fest verbunden sind,
- zur Ausrichtung der optischen Achse des Lichtleiters (7) in Bezug auf die *-* Längsachse der Absaugkanüle (3) eine Justierfläche (10) an dem ersten Justierstück (9) und eine Justierfläche (11) am Grundkörper (2) ausgebildet ist und beide Justierflächen (10, 11) so miteinander korrespondieren, daß der Lichtleiter (7) relativ zur Absaugkanüle (3) in axialer Richtung verschiebbar ist und die Ausrichtung in Abhängigkeit von der Verschieberichtung herstellbar oder aufhebbar ist, und
- zur Ausrichtung der optischen Achse des Lichtleiters (7) in Bezug auf das abstrahlungsseitige Ende (8) der Faser (6) eine Weitere Justierfläche (16) an dem ersten Justierstück (9) und eine weitere Justierfläche (17) an dem zweiten Justierstück (18) ausgebildet ist und beide weiteren Justierflächen (16, 17) so miteinander korrespondieren, daß der Lichtleiter (7) relativ zum abstrahlungsseitigen Ende (8) der Faser (6) in axialer Richtung verschiebbar ist und die Ausrichtung in Abhängigkeit von der Verschieberichtung herstellbar oder aufhebbar ist.

2. Handstück nach Anspruch 1, **dadurch gekennzeichnet, daß** die Justierfläche (10) an dem ersten Justierstück (9) als kreisrunde Zylinderaußenfläche und die Justierfläche (11) am Grundkörper (2) als kreisrunde Zylinderinnenfläche ausgebildet sind, die miteinander eine Gleitpassung bilden.

3. Handstück nach Anspruch 1, **dadurch gekennzeichnet, daß** die Justierfläche (10) an dem ersten Justierstück (9) als Kreiskegelaußenfläche und die Justierfläche (11) am Grundkörper (2) als Kreiskegelinnenfläche mit gegenseitig selbstzentrierender Eigenschaft ausgebildet sind und beide Kreiskegelflächen sich genau in der ausgerichteten Position der optischen Achse des Lichtleiters (7) in Kontakt befinden.

4. Handstück nach einem der Ansprüche 1 bis 3, **dadurch gekennzeichnet, daß** die weitere Justierfläche (16) an dem ersten Justierstück (9) als kreisrunde Zylinderaußenfläche und die Justierfläche (17) an dem zweiten Justierstück (18) als kreisrunde Zylinderinnenfläche ausgebildet sind, die miteinander eine Gleitpassung bilden.

5. Handstück nach einem der Ansprüche 1 bis 3, **dadurch gekennzeichnet, daß** die Justierfläche (16) an dem ersten Justierstück (9) als Kreiskegelaußenfläche und die Justierfläche (17) an dem zweiten Justierstück (18) als Kreiskegelinnenfläche mit gegenseitig selbstzentrierender Eigenschaft ausgebildet sind und beide Kreiskegelflächen sich genau in der ausgerichteten Position der optischen Achse des Lichtleiters (7) in Kontakt befinden.

6. Handstück nach Anspruch 3 oder 5, **dadurch gekennzeichnet, daß** die Verjüngungsrichtung der Kreiskegelflächen identisch ist mit der Richtung der Laserstrahlung, wodurch die Außenfläche durch Bewegung in axialer Richtung, jedoch entgegengesetzt zur Laserstrahlungsrichtung, von der Innenfläche lösbar ist.

7. Handstück nach einem der vorgenannten Ansprüche, **dadurch gekennzeichnet, daß** Anschläge zur Begrenzung oder zur Verhinderung der axialen Verschiebbarkeit des Lichtleiters (7) vorgesehen sind.

8. Handstück nach Anspruch 7, **dadurch gekennzeichnet, daß** die Anschläge als senkrecht zur Strahlungsrichtung ausgebildete Anschlagflächen (12,13) ausgebildet sind und mindestens eine Anschlagfläche fest in Relation zum Lichtleiter (7) und eine Anschlagfläche fest in Relation zur Absaugkanüle (3) angeordnet ist.

9. Handstück nach einem der vorgenannten Ansprüche, **dadurch gekennzeichnet, daß** Anschläge zur Verhinderung der axialen Verschiebbarkeit des Lichtleiters (7) vorgesehen sind, wobei Anschlagflächen des Lichtleiters (7) in axialer Richtung zwischen Anschlagflächen (12,13) der Absaugkanüle (3) und eines weiteren, mit dem Handstück verbundenen Teiles (14) unbeweglich eingeschlossen sind.

10. Handstück nach Anspruch 8 oder 9, **dadurch gekennzeichnet, daß** Anschläge zur Begrenzung der axialen Verschiebbarkeit vorgesehen sind, wobei Anschlagflächen des Lichtleiters mit begrenzter Bewegungsfreiheit in axialer Richtung zwisehen Anschlagflächen (12) der Absaugkanüle (3) bzw. eines weiteren, mit dem Handstück verbundenen Teiles (14) eingeschlossen sind.

11. Handstück nach Anspruch 10, **dadurch gekennzeichnet, daß** zwischen Anschlagflächen des Lichtleiters und Anschlagflächen (12) der Absaugkanüle (3) eine mechanische Feder und/oder ein elastischer Körper vorgesehen ist, wodurch eine axiale Verschiebung des Lichtleiters (7) durch Erhöhung der Federvorspannkraft und/oder durch Verformung des elastischen Körpers möglich ist.

12. Handstück nach Anspruch 8 bis 11, **dadurch gekennzeichnet, daß** die Anschlagflächen (12,13), zwischen denen die Anschlagflächen des Lichtleiters unbeweglich oder mit begrenzter Bewegungsfreiheit in axialer Richtung eingeschlossen sind, an verschiedenen, relativ zueinander frei beweglich Teilen (2,14) des Handstückes (1) ausgebildet sind.

13. Handstück nach einem der vorgenannten Ansprüche, **dadurch gekennzeichnet, daß** die dem Lichtleiter (7) zugeordnete justierfläche (16) an dem ersten Justierstück (9) und die der Faser (6) zugeordnete Justierfläche (17) an dem zweiten Justierstück (18) als kreisrunde Zylinderflächen und/oder als Kreiskegelflächen mit selbstzentrierenden Eigenschaften ausgebildet sind, wobei die Zylinder- bzw. Kegelachsen parallel zur optischen Achse des Lichtleiters (7) bzw. zur parallel zur optischen Achse der Faser (6) verlaufen.

14. Handstück nach einem der vorgenannten Ansprüche, **dadurch gekennzeichnet, daß** Mittel zur kraft- und/oder formschlüssigen Verbindung des Lichtleiters (7) mit dem abstrahlungsseitigen Ende (8) der Faser (6) bei zueinander ausgerichteten optischen Achsen von Lichtleiter (7) und Faser (6) vorgesehen sind.

15. Handstück nach Anspruch 14, **dadurch gekennzeichnet, daß** das abstrahlungsseitige Ende (8) der Faser (6) gemeinsam mit dem Lichtleiter (7) beweglich ist.

16. Handstück nach einem der vorgenannten Ansprüche, **dadurch gekennzeichnet, daß** zwischen aneinandergrenzenden Abschnitten der Anschlagflächen Dichtungselemente vorgesehen sind.

## Claims

1. Medical handpiece (1) for the transmission of energy from a laser radiation into biological tissue, the handpiece consisting of a body (2) that houses an optical fibre (6) for input conduction of the laser radiation, an optical waveguide (7) for radiating the laser energy into the tissue, and a aspirator tip (3), which is intended for removing the biological tissue minced under the action of the said energy, and inside of which the optical waveguide (7) runs, and
- which is provided with several adjusting surfaces (10,11,16,17) for aligning the optic axis of the optical waveguide (7) relative to both the longitudinal axis of the aspirator tip (3) and the optical axis of the radiation output end (8) of the fibre (6),
**characterized in that**
- there are firm mechanical connections between the aspirator tip (3) and the body (2), between the optical waveguide (7) and a first adjusting piece (9), and between the optical fibre (6) and a second adjusting piece (18), and **in that**,
- for the purpose of aligning the optical axis of the optical waveguide (7) relative to the longitudinal axis of the aspirator tip (3), one adjusting surface (10) is provided on the first adjusting piece (9) and another adjusting surface (11) is provided on the body (2), with these two adjusting surfaces (10, 11) corresponding with each other in such a way that the optical waveguide (7) can be displaced in axial direction relative to the aspirator tip (3) and the alignment can be done or undone depending on the direction of displacement, and **in that**
- for the purpose of aligning the optical axis of the optical waveguide (7) relative to the radiation output end (8) of the fibre (6), another adjusting surface (16) is provided on the first adjusting piece (9) and yet another adjusting surface (1 7) is provided on the second adjusting piece (18), with these two adjusting surfaces (16, 17) corresponding with each other in such a way that the optical waveguide (7) can be displaced in axial direction relative to the radiation output end (8) of the fibre (6) and alignment can be done or undone depending on the direction of displacement.

2. Handpiece as claimed in Claim 1, **characterized in that** the adjusting surface (10) provided on the first adjusting piece (9) is configured as an external circular cylinder surface and the adjusting surface (11) provided on the body (2) is configured as an internal circular cylinder surface, these two surfaces making up a sliding fit.

3. Handpiece as claimed in Claim 1, **characterized in that** the adjusting surface (10) provided on the first adjusting piece (9) is configured as an external surface of a circular cone and the adjusting surface (11) provided on the body (2) is configured as an internal surface of a circular cone, the two surfaces having the property of mutual self-centration and being in mutual contact exactly in the aligned position of the optical axis of the optical waveguide (7).

4. Handpiece as claimed in any of Claims 1 to 3, **characterized in that** the adjusting surface (16) provided on the first adjusting piece (9) is configured as an external circular cylinder surface and the adjusting surface (17) provided on the second adjusting piece (18) is configured as an internal circular cylinder surface, these two surfaces making up a sliding fit.

5. Handpiece as claimed in any of Claims 1 to 3, **characterized in that** the adjusting surface (16) provided on the first adjusting piece (9) is configured as an external surface of a circular cone and the adjusting surface (17) provided on the second adjusting piece (18) is configured as an internal surface of a circular cone, the two surfaces having the property of mutual self-centration and being in mutual contact exactly in the aligned position of the optical axis of the optical waveguide (7).

6. Handpiece as claimed in Claim 3 or 5, **characterized in that** the tapering direction of the circular cone surfaces is identical with the direction of the laser radiation, so that the external surface can be detached from the internal surface by a movement in axial direction but opposite to the direction of laser radiation.

7. Handpiece as claimed in any of the previous claims, **characterized in that** stops are provided for limiting or preventing the axial displacement of the optical waveguide (7).

8. Handpiece as claimed in Claim 7, **characterized in that** the stops are configured as stop surfaces (12,13) normal to the radiation direction, and **in that** at least one stop surface is arranged in a fixed position relative to the optical waveguide (7) and another stop surface is arranged in a fixed position relative to the aspirator tip (3).

9. Handpiece as claimed in any of the previous claims, **characterized in that** stops are provided for preventing axial displacement of the optical waveguide (7), with stop surfaces of the optical waveguide (7) being immovably included in axial direction between stop surfaces (12,13) of the aspirator tip (3) and of another piece (14) attached to the handpiece.

10. Handpiece as claimed in Claim 8 or 9, **characterized in that** stops are provided for preventing axial displacement, with stop surfaces of the optical waveguide (7) being included between stop surfaces (12) of the aspirator tip (3) and of another piece (14) attached to the handpiece, so that the stop surfaces of the optical waveguide (7) have a limited range of movement in axial direction.

11. Handpiece as claimed in Claim 10, **characterized in that** a mechanical spring and/or an elastic body is provided between stop surfaces of the optical waveguide and stop surfaces (12) of the aspirator tip (3), so that an axial displacement of the optical waveguide (7) is possible by an increase in the prestressing force of the spring and/or by a deformation of the elastic body.

12. Handpiece as claimed in Claims 8 to 11, **characterized in that** the stop surfaces (12,13), between which the stop surfaces of the optical waveguide are included either immovably or with a limited range of movement in axial direction, are provided on several parts (2,14) of the handpiece (1) that are freely movable relative to each other.

13. Handpiece as claimed in any of the previous claims, **characterized in that** the adjusting surface (16) provided on the first adjusting piece (9), which is assigned to the optical waveguide (7), and the adjusting surface (1 7) provided on the second adjusting piece (18), which is assigned to the fibre (6), are configured as circular cylinder surfaces and/or as circular cone surfaces with self-centration properties, with the cylinder and/or cone axes are parallel to the optical axis of the optical waveguide (7) or parallel to the optical axis of the fibre (6), respectively.

14. Handpiece as claimed in any of the previous claims, **characterized in that** means are provided for non-positive and/or positive connection between the optical waveguide (7) and the radiation output end (8) of the fibre (6), with the optical axes of the optical waveguide (7) and the fibre (6) being in alignment.

15. Handpiece as claimed in Claim 14, **characterized in that** the radiation output end (8) of the fibre (6) is movable jointly with the optical waveguide (7).

16. Handpiece as claimed in any of the previous claims, **characterized in that** sealing elements are provided between adjacent segments of the stop surfaces.

## Revendications

1. Poignée médicale (1) pour transmettre de l'énergie d'un rayonnement laser dans un tissu biologique, dans laquelle sont prévus, dans un corps de base, (2) une fibre optique (6) pour guider le rayonnement laser, un guide optique (7) pour le rayonnement de l'énergie laser dans le tissu ainsi qu'une canule d'aspiration (3) pour le tissu biologique fragmenté sous l'effet de l'énergie, dans laquelle s'étend le guide optique (7), et
pour l'orientation de l'axe optique du guide optique (7) aussi bien par rapport à l'axe longitudinal de la canule d'aspiration (3) que par rapport à l'axe optique de l'extrémité (8) côté rayonnement de la fibre (6), sont prévues plusieurs surfaces d'ajustage (10, 11, 16, 17),
**caractérisée en ce que**
la canule d'aspiration (3) avec le corps de base (2), le guide optique (7) avec une première pièce d'ajustage (9) et la fibre optique (6) avec une deuxième pièce d'ajustage (18) sont reliés mécaniquement de manière fixe,
pour l'orientation de l'axe optique du guide optique (7) par rapport à l'axe longitudinal de la canule d'aspiration (3), une surface d'ajustage (10) est réalisée sur la première pièce d'ajustage (9) et une surface d'ajustage (11) sur le corps de base (2), et les deux surfaces d'ajustage (10, 11) correspondent l'une à l'autre de manière que le guide optique (7) puisse coulisser dans la direction axiale par rapport à la canule d'aspiration (3), et l'orientation peut être réalisée ou supprimée en fonction du sens de coulissement, et
pour l'orientation de l'axe optique du guide optique (7) par rapport à l'extrémité (8) côté rayonnement de la fibre (6), une autre surface d'ajustage (16) est réalisée sur la première pièce d'ajustage (9) et une autre surface d'ajustage (17) sur la deuxième pièce d'ajustage (18), et les deux autres surfaces d'ajustage (16, 17) correspondent l'une à l'autre de manière que le guide optique (7) puisse coulisser dans la direction axiale par rapport à l'extrémité (8) côté rayonnement de la fibre (6), et l'orientation peut être réalisée ou supprimée en fonction du sens de coulissement.

2. Poignée selon la revendication 1, **caractérisée en ce que** la surface d'ajustage (10) sur la première pièce d'ajustage (9) est réalisée en tant que surface extérieure circulaire d'un cylindre et la surface d'ajustage (11) sur le corps de base (2) en tant que surface intérieure circulaire d'un cylindre, lesquelles surfaces forment un ajustement glissant.

3. Poignée selon la revendication 1, **caractérisée en ce que** la surface d'ajustage (10) sur la première pièce d'ajustage (9) est réalisée en tant que surface extérieure d'un cône de révolution et la surface d'ajustage (11) sur le corps de base (2) en tant que surface intérieure d'un cône de révolution avec propriété d'auto-centrage réciproque, et les deux surfaces du cône de révolution se trouvent en contact exactement dans la position orientée de l'axe optique du guide optique (7).

4. Poignée selon l'une des revendications 1 à 3, **caractérisée en ce que** l'autre surface d'ajustage (16) sur la première pièce d'ajustage (9) est réalisée en tant que surface extérieure circulaire d'un cylindre et la surface d'ajustage (17) sur la deuxième pièce d'ajustage (18) en tant que surface intérieure circulaire d'un cylindre, lesquelles surfaces forment entre elles un ajustement glissant.

5. Poignée selon l'une des revendications 1 à 3, **caractérisée en ce que** la surface d'ajustage (16) sur la première pièce d'ajustage (9) est réalisée en tant que surface extérieure d'un cône de révolution et la surface d'ajustage (17) sur la deuxième pièce d'ajustage (18) en tant que surface intérieure d'un cône de révolution avec propriété d'autocentrage réciproque, et les deux surfaces du cône de révolution se trouvent en contact exactement dans la position orientée de l'axe optique du guide optique (7).

6. Poignée selon la revendication 3 ou 5, **caractérisée en ce que** le sens de rétrécissement des surfaces du cône de révolution est identique au sens du rayonnement laser, ce qui fait que la surface extérieure peut être séparée de la surface intérieure par mouvement dans la direction axiale, mais dans un sens opposé au sens du rayonnement laser.

7. Poignée selon l'une des revendications précédentes, **caractérisée en ce que** des butées sont prévues pour limiter ou empêcher la possibilité de coulissement axial du guide optique (7).

8. Poignée selon la revendication 7, **caractérisée en ce que** les butées sont réalisées en tant que surfaces de butée (12, 13) réalisées perpendiculairement à la direction du rayonnement, et au moins une surface de butée est disposée de manière fixe par rapport au guide optique (7) et une surface de butée est disposée de manière fixe par rapport à la canule d'aspiration (3).

9. Poignée selon l'une des revendications précédentes, **caractérisée en ce que** sont prévues des butées pour empêcher la possibilité de coulissement axial du guide optique (7), des surfaces de butée du guide optique (7) étant immobilisées dans la direction axiale entre des surfaces de butée (12, 13) de la canule d'aspiration (3) et une autre partie (14) reliée à la poignée.

10. Poignée selon la revendication 8 ou 9, **caractérisée en ce que** sont prévues des butées pour limiter la possibilité de coulissement axial, des surfaces de butée du guide optique avec liberté de mouvement limitée dans la direction axiale étant enfermées entre des surfaces de butée (12) de la canule d'aspiration (3) ou une autre partie (14) reliée à la poignée.

11. Poignée selon la revendication 10, **caractérisée en ce qu'**entre des surfaces de butée du guide optique et des surfaces de butée (12) de la canule d'aspiration (3) est prévu un ressort mécanique et/ou un corps élastique, ce qui fait qu'un coulissement axial du guide optique (7) est possible par augmentation de la force de précontrainte du ressort et/ou par déformation du corps élastique.

12. Poignée selon les revendications 8 à 11, **caractérisée en ce que** les surfaces de butée (12, 13), entre lesquelles les surfaces de butée du guide optique sont enfermées sans possibilité de mouvement ou avec une liberté de mouvement limitée dans la direction axiale, sont réalisées sur différentes parties (2, 14), librement déplaçables l'une par rapport à l'autre, de la poignée (1).

13. Poignée selon l'une des revendications précédentes, **caractérisée en ce que** la surface d'ajustage (16), associée au guide optique (7), sur la première pièce d'ajustage (9) et la surface d'ajustage (17), associée à la fibre (6), sur la deuxième pièce d'ajustage (18), sont réalisées en tant que surfaces circulaires d'un cylindre et/ou en tant que surfaces d'un cône de révolution avec propriétés d'auto-centrage, les axes du cylindre ou du cône s'étendant parallèlement à l'axe optique du guide optique (7) ou parallèlement à l'axe optique de la fibre (6).

14. Poignée selon l'une des revendications précédentes, **caractérisée en ce que** sont prévus des moyens pour la liaison à force et/ou par complémentarité de formes du guide optique (7) avec l'extrémité (8) côté rayonnement de la fibre (6), lorsque les axes optiques du guide optique (7) et de la fibre (6) sont orientés l'un par rapport à l'autre.

15. Poignée selon la revendication 14, **caractérisée en ce que** l'extrémité (8) côté rayonnement de la fibre (6) est déplaçable avec le guide optique (7).

16. Poignée selon l'une des revendications précédentes, **caractérisée en ce que** des éléments d'étanchéité sont prévus entre des parties adjacentes les unes aux autres des surfaces de butée.
